# EUROPÄISCHE PATENTSCHRIFT

(11) **EP 3 807 051 B1**
(45) Veröffentlichungstag und Bekanntmachung des Hinweises auf die Patenterteilung: **12.03.2025**
(21) Anmeldenummer: 19731942.9
(22) Anmeldetag: 14.06.2019
(51) Int. Cl.: B25B 15/00, B25B 23/14, A61B 17/88, B25B 23/142

(54) **DREHMOMENTBEGRENZUNGSEINRICHTUNG UND VERFAHREN ZU DESSEN KALIBRIERUNG**
TORQUE-LIMITING DEVICE AND METHOD FOR THE CALIBRATION THEREOF
DISPOSITIF LIMITEUR DE COUPLE ET SON PROCÉDÉ D'ÉTALONNAGE

(30) Priorität: 15.06.2018 DE 102018114431
(43) Veröffentlichungstag der Anmeldung: 21.04.2021
(73) Patentinhaber: Wera Werkzeuge GmbH, 42349 Wuppertal (DE)
(72) Erfinder: ABEL, Michael, 42477 Radevormwald (DE)
(74) Vertreter: Grundmann, Dirk
(86) Internationale Anmeldenummer: PCT/EP2019/065691
(87) Internationale Veröffentlichungsnummer: WO 2019/238932

(56) Entgegenhaltungen:
- EP-A2- 1 092 510
- WO-A1-2014/035496
- DE-A1- 102016 013 174

## Beschreibung

### Gebiet der Technik

Die Erfindung betrifft eine Drehmomentbegrenzungseinrichtung mit zwei sich als Folge einer Schrägflankenanlage bei einer Relativdrehung um eine Achse in Richtung nämlicher Achse relativ zueinander gegen die Rückstellkraft eines sich an einer bei einer Kalibrierung eines Grenzdrehmomentes lageeingestellten Schulter eines Widerlagers abstützenden Federelementes verlagerbaren Drehmomentübertragungskörpern.

Die Erfindung betrifft darüber hinaus ein Kalibrierverfahren zum Kalibrieren einer Drehmomentübertragungseinrichtung mit zwei sich als Folge einer Schrägflankenanlage bei einer Relativdrehung um eine Achse in Richtung nämlicher Achse relativ zueinander gegen die Rückstellkraft eines sich an einer bei einer Kalibrierung eines Grenzdrehmomentes lageeingestellten Schulter eines Widerlagers abstützenden Federelementes verlagerbaren Drehmomentübertragungskörpern.

Die Erfindung betrifft darüber hinaus eine Mehrzahl von Schraubwerkzeugen, die jeweils eine gleichgestaltete Drehmomentbegrenzungseinrichtung aufweisen.

### Stand der Technik

Eine gattungsgemäße Drehmomentbegrenzungseinrichtung wird in der DE 10 2014 212 222 A1 beschrieben. Ein aus Stahl gefertigter Schaft weist an seinem freien Ende ein Schraubwerkzeugprofil auf. Das gegenüberliegende Ende des Schaftes besitzt ein Außengewinde, auf dem eine Mutter aufgeschraubt ist. An einem Vorsprung des Schaftes stützt sich ein erster, drehfest und axialfest mit dem Schaft verbundener Drehmomentübertragungskörper ab. Der Drehmomentübertragungskörper besitzt Drehmomentübertragungszähne mit sägezahnartig angeordneten Zahnflanken. An einer schräg verlaufenden Zahnflanke eines Drehmomentübertragungszahnes stützt sich eine schräg verlaufende Zahnflanke eines zweiten Drehmomentübertragungskörpers ab, der in Achsrichtung des Schaftes von einem Federelement beaufschlagt wird. Das Federelement stützt sich an der auf das Außengewinde des Schaftes aufgeschraubten Mutter ab. Diese Drehmomentbegrenzungseinrichtung kann in ein Schraubwerkzeug eingebaut werden, beispielsweise kann die Drehmomentübertragungseinrichtung wie in der EP 1 631 418 B1 gezeigt, in eine Griffhöhlung eines Schraubendrehergriffs eingesetzt werden. Die Drehmomentbegrenzungseinrichtung kann mittels der Mutter kalibriert werden. Durch Verdrehen der Mutter ändert sich die Vorspannung des Federelementes, mit der der zweite Drehmomentübertragungskörper in Achsrichtung beaufschlagt wird. Wird zwischen den beiden Drehmomentübertragungskörpern ein Drehmoment erzeugt, so führt dies zu einer Relativverdrehung des zweiten Drehmomentübertragungskörpers, der axial beweglich und drehbeweglich dem Schaft zugeordnet ist, gegenüber dem ersten Drehmomentübertragungskörper, wobei bei dieser Relativdrehung die beiden Schrägflanken aneinander abgleiten mit der Folge, dass sich die beiden Drehmomentübertragungskörper axial voneinander entfernen. Im Zuge dieser Axialverlagerung des zweiten Drehmomentübertragungskörpers wird das Federelement so weit gespannt, bis der Drehmomentübertragungszahn des zweiten Drehmomentübertragungskörpers den Drehmomentübertragungszahn des ersten Drehmomentübertragungskörpers überläuft. Die dann erreichte Federvorspannung entspricht einem Auslösedrehmoment, bei dessen Erreichen kein Drehmoment mehr vom einen Drehmomentübertragungskörper auf den anderen Drehmomentübertragungskörper übertragen wird, weil sich die beiden Drehmomentübertragungskörper bei Überschreiten dieses Grenzdrehmomentes relativ zueinander verdrehen.

Beim Stand der Technik wird dieses Auslösedrehmoment durch Verdrehen der Mutter eingestellt.

Die WO 2014/035496 A1 offenbart eine Drehmomentbegrenzungseinrichtung mit einem ein Federelement beaufschlagendes Widerlager, das materialeinheitlich von einem an einem Schaft befestigten Abstützelement gebildet ist. Der Drehmomentbegrenzungswert wird durch Austausch des Widerlagers und/oder des Federelementes geändert.

Bei der EP 1 092 510 A2 wird die Vorspannung des Federelementes durch die Auswahl einzelner Bauteile, wie eines Federelementes und Unterlegscheiben, eingestellt.

Die DE 10 2016 013174 A1 offenbart ebenfalls eine fest eingestellte Drehmomentbegrenzung, wobei die Einstellung des Drehmomentbegrenzung durch Vorgabe der Relativausrichtung zweier Gehäusebauteile, zwischen denen das Federelement angeordnet ist, erfolgt.

Im Stand der Technik gibt es darüber hinaus Drehmomentbegrenzungseinrichtungen, die keine Kalibrierung des Auslösedrehmomentes ermöglichen. Bei der Herstellung derartiger Drehmomentbegrenzungseinrichtungen werden Federelemente verwendet, deren Federkonstante in einem engen Toleranzbereich liegt. Eine erstmalige Kalibrierung kann hier durch die Verwendung von Unterlegscheiben durchgeführt werden. Ein derartiges Schraubwerkzeug wird in der EP 1 092 510 B1 beschrieben.

Die DE 10 2016 013 174 A1 beschreibt ein Kleinst-Drehmomentwerkzeug mit einem flachen Gehäuse, aus dem ein im Gehäuse gelagerter Schaft heraustritt. Auf dem Schaft sind zwei Drehmomentübertragungskörper gelagert, wobei ein Drehmomentübertragungskörper mit einer Druckfeder gegen den anderen Drehmomentübertragungskörper beaufschlagt ist. Die Kraft, mit der die Druckfeder den Drehmomentübertragungskörper beaufschlagt, wird bei der Montage der beiden Gehäuseteile festgelegt. Die beiden Gehäuseteile werden mit einer vorgegebenen Kraft gegeneinander beaufschlagt und in diesem Zustand miteinander verschweißt.

### Zusammenfassung der Erfindung

Der Erfindung liegt die Aufgabe zugrunde, ein mit einfachen Mitteln kalibriertes Schraubwerkzeug anzugeben und Maßnahmen vorzuschlagen, mit denen das kalibrierte Auslösedrehmoment nicht nachträglich verändert werden kann sowie ein Verfahren anzugeben, mit dem eine derartige Kalibrierung möglich ist.

Gelöst wird die Aufgabe durch die in den Ansprüchen angegebene Erfindung, wobei die Unteransprüche nicht nur vorteilhafte Weiterbildungen der nebengeordneten Ansprüche, sondern auch eigenständige Lösungen der Aufgabe darstellen.

Zunächst und im Wesentlichen wird vorgeschlagen, dass die Kalibrierung der Drehmomentbegrenzungseinrichtung nicht durch das Messen eines von den Drehmomentübertragungskörpern übertragbaren Auslösemomentes beziehungsweise Grenzdrehmomentes erfolgt, sondern dass das Federelement unter Verwendung einer vorbestimmten Kraft vorgespannt wird, wobei durch das Vorspannen des Federelementes eine axiale Lage des Widerlagers festgelegt wird, in der das Widerlager irreversibel fixiert wird. Indem das Widerlager irreversibel fixiert wird, kann ein einmal eingestelltes Auslösedrehmoment nicht mehr verändert werden. Dies erhöht die Manipulationssicherheit eines auf einen bestimmten Drehmomentwert kalibriertes Drehmomentwerkzeug. Das Fixieren des Widerlagers in der durch Aufbringen einer vorbestimmten Kraft gefundenen axialen Lage erfolgt bevorzugt, während das Federelement noch mit der vorbestimmten Kraft beaufschlagt wird. Dies kann beispielsweise dadurch erfolgen, dass das Federelement oder ein auf dem Federelement aufliegendes Zwischenglied, wie beispielsweise eine Unterlegscheibe, mit einer vorbestimmten Gewichtskraft beaufschlagt wird. Hierzu können Normgewichte verwendet werden. Da die Federelemente, die bevorzugt von Wendelgangdruckfedern ausgebildet sind, toleranzbehaftete Längen und toleranzbehaftete Federkonstanten aufweisen, bekommen die vorgespannten Federelemente eine individuelle axiale Länge, in der die mit der vorbestimmten Kraft beaufschlagte und komprimierte, vorzugsweise Wendelgang-Druckfeder lagefixiert wird. Die axiale Lage des Widerlagers kann auf verschiedene Weise irreversibel festgelegt werden, beispielsweise durch eine formschlüssige Verbindung, eine kraftschlüssige Verbindung oder eine stoffschlüssige Verbindung zu einem Schaft oder zu einem anderen Element der Drehmomentbegrenzungseinrichtung, die axialfest mit einem Drehmomentübertragungskörper verbunden ist. Beispielsweise ist es möglich, einen Widerlagerkörper über einen Schweißpunkt oder eine Lötverbindung mit einem freien Ende eines Schaftes zu verbinden. Die Schulter kann aber auch von einem Schweißpunkt selbst ausgebildet sein. Es ist ebenfalls möglich, einen Widerlagerkörper über eine Klebeverbindung mit dem Schaft zu verbinden. Auch formschlüssige Verbindungen eines Widerlagerkörpers mit dem Schaft sind möglich, beispielsweise durch Schaffen einer Bohrung und Hindurchstecken eines Splintes, wobei die räumliche Zuordnung des Widerlagerkörpers beispielsweise zum Schaft der Drehmomentbegrenzungseinrichtung bei vorgespanntem Federelement erfolgt. Anstelle des Splintes kann auch ein Keil verwendet werden, der in einen Längsschlitz des Schaftes eingebracht wird und dort irreversibel fixiert wird. Ein aus dem Stand der Technik her bekannte Mutter kann beispielsweise über einen Schweißpunkt oder dergleichen irreversibel drehfixiert werden. Bei einer bevorzugten Ausgestaltung der erfindungsgemäßen Drehmomentbegrenzungseinrichtung wird das Widerlager durch eine plastische Verformung erzeugt. Es ist insbesondere vorgesehen, dass ein freies Ende eines Schaftes plastisch verformt wird, so dass sich eine Schulter ausbildet, an der sich das Federelement, bevorzugt die aus Stahl gefertigte Wendelgang-Druckfeder, gegebenenfalls unter Zwischenlage eines Zwischengliedes, beispielsweise einer Unterlegscheibe, abstützt. Das andere Ende des Federelementes kann sich an einem axial gegenüber dem Schaft beweglichen und gegenüber dem Schaft drehbaren Drehmomentübertragungskörper abstützen, welcher Schrägflanken ausbildet, die an Schrägflanken eines weiteren Drehmomentübertragungskörpers anliegen, der axial- und drehfest mit dem Schaft verbunden ist. Es kann ein zweites Zwischenglied, ebenfalls in Form einer Unterlegscheibe, vorgesehen sein, welches dem zweiten Drehmomentübertragungskörper zugeordnet ist. Das Federelement kann sich somit mit beiden Enden jeweils an einer Unterlegscheibe abstützen. Der erste Drehmomentübertragungskörper, der drehfest mit dem Schaft verbunden ist, kann auf ein Einsatzstück aufgesetzt sein. Das Einsatzstück kann in einer Höhlung des zweiten Drehmomentübertragungskörper stecken. Bei dem Einsatzstück handelt es sich bevorzugt um eine Sechskant-Mitnehmerscheibe, die einen Außensechskant aufweist und eine Innensechskantöffnung. Die Höhlung des Drehmomentübertragungskörpers besitzt ein Innensechskantprofil, in dem das Sechskant-Außenprofil der Sechskant-Mitnehmerscheibe formschlüssig einliegt. Die Flanken des Innensechskantprofils der Sechskant-Mitnehmerscheibe liegen an einem Sechskantabschnitt des Schaftes an. Mit der Sechskant-Mitnehmerscheibe vergrößern sich die vom Schaft auf den zweiten Drehmomentübertragungskörper übertragbaren Drehmomente. Um die beiden Drehmomentübertragungskörper beim Aufbringen eines Relativdrehmomentes relativ zueinander in Achsrichtung zu verlagern, kann eine Hubeinrichtung vorgesehen sein. Diese Hubeinrichtung kann von Drehmomentübertragungszähnen ausgebildet sein, die Schrägflanken ausbilden, die aneinanderliegen und bei der Relativverlagerung aneinander abgleiten. Es sind auch alternative Ausgestaltungen einer Hubeinrichtung möglich. So können Kugeln vorgesehen sein, die in Kugelaufnahmeöffnungen liegen, wobei jeder der beiden Drehmomentübertragungskörper ein oder mehrere Kugelaufnahmeöffnungen aufweisen.

Bei dem erfindungsgemäßen Verfahren lässt sich eine Vielzahl von im Wesentlichen baugleichen Drehmomentbegrenzungseinrichtungen in einfacher Weise kalibrieren. Hierzu werden jeweils Drehmomentübertragungskörper verwendet, die Drehmomentübertragungszähne aufweisen, welche schräg verlaufende Zahnflanken aufweisen, die im nicht drehmomentbeaufschlagten Zustand in einer Flächenanlage zueinander liegen. Es kann aber auch vorgesehen sein, dass lediglich ein Drehmomentübertragungskörper eine oder mehrere Schrägflanken aufweist, an dem beispielsweise eine Rundung oder eine andere Randkante des anderen Drehmomentübertragungskörpers anliegt. Wesentlich ist, dass die Steigung der Schrägflanke des zumindest einen eine Schrägflanke ausbildenden Drehmomentübertragungskörpers in einem engen Toleranzfeld liegt. Diese fertigungstechnischen Randbedingungen sind in ausreichender Weise realisierbar, wenn es sich bei den Drehmomentübertragungskörpern um Kunststoff- oder Metall-Spritzgussteile, Sinterteile, Frästeile oder Feingussteile handelt. Die Schräge und der axiale Abstand der Schrägfläche zur Drehachse definiert das Auslösemoment bei einer vorgegebenen Kraft.

Bei dem erfindungsgemäßen Kalibrierverfahren werden Federelemente verwendet, die toleranzbedingt voneinander verschiedene Längen und voneinander verschiedene Federkonstanten aufweisen. Erfindungsgemäß erfolgt die Kalibrierung der Drehmomentbegrenzungseinrichtung bei deren Zusammenbau, wobei zunächst die beiden Drehmomentübertragungskörper an einem Schaft montiert werden, bedarfsweise eine Unterlegscheibe auf den zuoberst liegenden Drehmomentübertragungskörper aufgelegt wird und anschließend das Federelement, bei dem es sich um eine Stahlfeder handeln kann, auf den zuoberst liegenden Drehmomentübertragungskörper aufgesetzt wird. Optional kann auf das nach oben weisende Ende des Federelementes ein Zwischenglied, beispielsweise eine Unterlegscheibe, aufgelegt werden. Das Federelement beziehungsweise das Zwischenglied wird von einem Abschnitt des Schaftes, an dem sich der untere Drehmomentübertragungskörper abstützt, überragt. In einem Kalibrierschritt wird jetzt das Federelement von oben her mit einer vordefinierten Kraft, beispielsweise mit der Gewichtskraft eines Normgewichtes, kraftbeaufschlagt. Dies hat zur Folge, dass das Federelement auf eine individuelle Länge komprimiert wird. Das Federelement wird dann in dieser kalibrierten Länge lagefixiert. Dies erfolgt durch das Positionieren einer Schulter eines Widerlagers, wobei insbesondere vorgesehen ist, dass die Schulter beim Lagefixieren des nach oben weisenden Endes des Federelementes durch eine plastische Verformung erzeugt wird. Beispielsweise können zwei Pressbacken vorgesehen sein, die das freie des Schaftes in Radialrichtung beaufschlagen, so dass es querschnittsvermindert wird und sich quer zur Kraftbeaufschlagung sich erstreckende Schultern ausbilden, an denen sich das Federelement oder das Zwischenglied abstützen können. Bei dieser Variante der Erfindung wird mit der vorbestimmten Kraft eine Grundlänge des Federelementes eingestellt, die beim Aufbringen eines Drehmomentes auf die beiden Drehmomentübertragungskörper weiter vermindert wird bis zu einer Auslöselänge, bei der der Drehmomentübertragungszahn des drehbeweglichen Drehmomentübertragungskörpers den Drehmomentübertragungszahn des drehfest mit dem Schaft verbundenen Drehmomentübertragungskörpers überläuft. In einer Variante der Erfindung kann aber auch vorgesehen sein, dass mit der vorbestimmten Kraft die Federvorspannung eingestellt wird, die zum Auslösedrehmoment korrespondiert. Bei dieser zweiten Verfahrensvariante wird eine größere vorbestimmte Kraft verwendet als bei der ersten Verfahrensvariante. Während bei der ersten Verfahrensvariante das Widerlager exakt an der Position erzeugt oder fixiert wird, an der sich das Federelement oder das Zwischenglied abstützen soll, kann das Widerlager bei der zweiten Verfahrensvariante in einem axialen Abstand zum oberen Ende des Federelementes beziehungsweise des Zwischengliedes erzeugt werden, wobei der Abstand zwischen dem oberen Ende des Federelementes beziehungsweise dem oberen Ende des Zwischengliedes vom Widerlager beziehungsweise der das Widerlager ausbildenden Schulter dem axialen Verlagerungsweg des beweglichen Drehmomentübertragungskörpers entspricht, also beispielsweise der Höhe eines Drehmomentübertragungszahnes. Alternativ dazu können bei der Lagefixierung des Widerlagers die beiden Drehmomentübertragungskörper auch in eine Drehstellung gebracht werden, in der sich die Drehmomentübertragungszähne aneinander abstützen, die Drehmomentübertragungskörper also eine Beabstandungslage erhalten, die beim Überschreiten des Auslösedrehmomentes erreicht wird. In dieser Stellung der Drehmomentübertragungskörper kann dann das sich am oberen Drehmomentübertragungskörper abstützende Federelement durch Aufbringen einer vorbestimmten Kraft längeneingestellt werden und in dieser Längeneinstellung das Widerlager geschaffen oder lagefixiert wird.

Die Erfindung betrifft somit eine Drehmomentbegrenzungseinrichtung, bei der ein Federelement zwischen zwei Lagerelementen eingespannt ist, von denen eines ein Lager und das andere ein Widerlager ausbildet. Sowohl Lager als auch Widerlager sind bevorzugt irreversibel an einem Schaft befestigt. Gemäß einer Weiterbildung der Erfindung wird vorgeschlagen, dass das Widerlager von einem Zwischenglied beziehungsweise Abstützelement ausgebildet ist. Das Abstützelement kann einen tellerförmigen Abschnitt aufweisen, an dem sich unmittelbar einer der Drehmomentübertragungskörper oder die Druckfeder abstützt. Das Abstützelement ist bevorzugt einhergehend mit einer plastischen Verformung am Schaft fixiert. Dies erfolgt in der zuvor beschriebenen Weise, indem bei der Kalibrierung das Abstützelement in Achsrichtung des Schaftes mit einer vorgegebenen Kraft beaufschlagt wird. Dabei können die ineinandergreifenden Zähne der Drehmomentübertragungskörper Kopf auf Kopf stehen, so dass die Kraft eingestellt wird, die beim Auslösen des Grenzdrehmomentes überwunden werden muss. In einer bevorzugten Ausgestaltung besitzt das Abstützelement, welches aus einem weicheren Material gefertigt sein kann, als der bevorzugt gehärtete Schaft, der auch das Schraubwerkzeug ausbilden kann, einen Hals, der vom tellerförmigen Abschnitt abragt. Der Innendurchmesser des sich in Axialrichtung erstreckenden Halses ist bevorzugt nur wenig größer, als der Außendurchmesser des Schaftes, so dass das Abstützelement auf den Schaft aufgesteckt werden kann. In einer bevorzugten Ausgestaltung besitzt das Ende des Schaftes eine Aussparung, die insbesondere eine Konusfläche aufweist. Die Aussparung kann auch eine Stufe aufweisen. Bevorzugt grenzt die Konusfläche an eine ringförmige Stufe. In diese Aussparung wird bei der irreversiblen Fixierung des Abstützelementes am Schaft Material des Halses eingepresst. Dies erfolgt mittels eines Presswerkzeuges. Das Aufpressen erfolgt während das Abstützelement in Achsrichtung mit der vorgegebenen Kraft kraftbeaufschlagt ist. Das Material des Abstützelementes fließt in die Aussparung des Schaftes, um dieserart eine Formschlussverbindung zu schaffen. Die Aussparung kann sich um den gesamten Schaft herum erstrecken. In einer Weiterbildung der Erfindung kann vorgesehen sein, dass zwischen einem Ende des Federelementes und dem Abstützelement eine Gleitlagerscheibe angeordnet ist. Die Gleitlagerscheibe kann von einer Unterlegscheibe gebildet sein. Dadurch wird die Anlagefläche des Federelementes gegenüber dem Abstützelement rotatorisch entkoppelt. Es können mehrere derartige Gleitlagerscheiben, also beispielsweise Unterlegscheiben, installiert werden. Sie können dann paketartig übereinander angeordnet sein, so dass sich der Schaft mitsamt dem am Schaft befestigten Abstützelement gegenüber dem Federelement drehen kann. Die Gleitlagerscheibe kann aus einem gleitlagertypischen Werkstoff, beispielsweise Stahl, Kunststoff, Keramik, Graphit oder aus Sinterwerkstoffen gefertigt werden. Es ist auch die Wahl von Nichteisenlegierungen vorgesehen. Eine derartige Gleitlagerscheibe kann auch bei solchen Ausführungsformen verwendet werden, bei denen der Schaft plastisch verformt wird, um eine widerlagernde Funktion auszuüben.

### Kurze Beschreibung der Zeichnungen

Ausführungsbeispiele der Erfindung werden nachfolgend anhand beigefügter Zeichnungen erläutert. Es zeigen:
- Fig. 1: eine in einen Schraubwerkzeuggriff eingesetzte Drehmomentübertragungseinrichtung,
- Fig. 2: die Draufsicht auf die Drehmomentübertragungseinrichtung gemäß Figur 1,
- Fig. 3: den Schnitt gemäß der Linie III-III vergrößert, wobei zwei Drehmomentübertragungskörper 1, 2 ihre Grundstellung einnehmen und von einem vorgespannten Federelement 8 beaufschlagt werden,
- Fig. 4: eine Darstellung gemäß Figur 3, wobei jedoch die beiden Drehmomentübertragungskörper 1, 2 gegeneinander drehverlagert worden sind, bis das Federelement 8 eine minimale Länge L2 erhält,
- Fig. 5: eine Darstellung ähnlich der Figur 3, jedoch in einer Montagestellung, in der das unkomprimierte Federelement 8 eine Neutrallänge L0 aufweist,
- Fig. 6: eine Darstellung gemäß Figur 5 nach Beaufschlagung des Federelementes 8 mit einer vorbestimmten Kraft F1, bei der das Federelement 8 auf die Kalibrierungslänge L1 zusammengedrückt wird,
- Fig. 7: eine Folgedarstellung, in der das obere Ende des Federelementes 8 relativ gegenüber dem Schaft 3 lagefixiert wird,

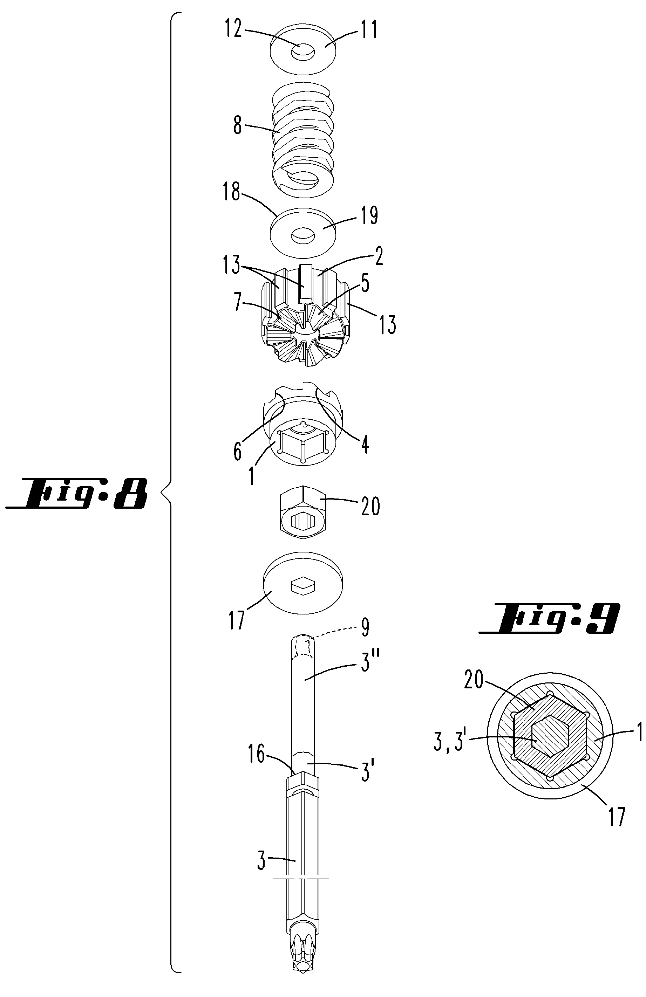

- Fig. 8: eine Explosionsdarstellung,
- Fig. 9: einen Schnitt gemäß der Linie IX-IX in Figur 4,
- Fig. 10: ein weiteres Ausführungsbeispiel der Erfindung in einer Darstellung gemäß Figur 3,
- Fig. 11: das in der Figur 10 dargestellte Ausführungsbeispiel nach Fixierung des Abstützelementes 21 am Schaft 3,
- Fig. 12: eine Darstellung gemäß Figur 11, jedoch mit einem Federelement 8, das eine andere Federcharakteristik aufweist, wobei das Abstützelement in einer anderen Achslage zum Schaft 3 befestigt ist,
- Fig. 13: eine Darstellung eines weiteren Ausführungsbeispiels gemäß Figur 10.

### Beschreibung der Ausführungsformen

Die Drehmomentbegrenzungseinrichtung besitzt einen aus Stahl gefertigten Schaft 3, der einen Vorsprung oder eine Stufe 16 aufweist, an der sich ein erster Drehmomentübertragungskörper 1 abstützt. Der beispielsweise als Kunststoff-Spritzgußteil, Metall-Spritzgußteil, Frästeil, Feingußteil gefertigte Drehmomentübertragungskörper 1 ist drehfest mit dem Schaft 3 verbunden und ändert seine axiale Lage gegenüber dem Schaft 3 nicht. Der erste Drehmomentübertragungskörper 1 besitzt einen Drehmomentübertragungszahn mit einer Schrägflanke 4 und einer dieser gegenüberliegenden Steilflanke 6. Beim Ausführungsbeispiel stützt sich an der Stufe 16 eine Unterlegscheibe 17 ab, auf der sich wiederum der Drehmomentübertragungskörper 1 abstützt. Der Drehmomentübertragungskörper 1 besitzt eine zur Unterlegscheibe 17 offene Höhlung mit einem Sechskantinnenprofil, in die eine aus Stahl gefertigte Sechskant-Mitnahmescheibe 20 eingesetzt ist. Diese besitzt ein Mehrkant-Innenprofil, das von einem Mehrkant-Außenprofilabschnitt 3' des Schaftes 3 ausgefüllt ist.

Ein zweiter Drehmomentübertragungskörper 2, der ebenfalls als Kunststoff-Spritzgußteil oder Metall-Spritzgußteil, Frästeil, Feingußteil oder Sinterteil ausgebildet sein kann, kann sich gegenüber dem ersten Drehmomentübertragungskörper 1 in Achsrichtung und in Umfangsrichtung, bezogen auf die Achse A, verlagern. Der zweite Drehmomentübertragungskörper 2 kann drehfest mit einem Griff verbunden sein, der beispielsweise eine Höhlung aufweist, in der die Drehmomentübertragungseinrichtung steckt. Hierzu kann der zweite Drehmomentübertragungskörper 2 sich in Achsrichtung erstreckende Umfangsnuten beziehungsweise Umfangsrippen 13 aufweisen, die in ein Gegenprofil der Höhlung des Griffes eingreifen. Der zweite Drehmomentübertragungskörper 2 besitzt ebenfalls Drehmomentübertragungszähne. Die Lücken zwischen den beiden Drehmomentübertragungszähnen bilden eine zweite Schrägflanke 5 und eine zweite Steilflanke 7 aus. Die zweite Schrägflanke 5 liegt in Flächenanlage an der ersten Schrägflanke 4. Die zweite Steilflanke 7 liegt in Flächenanlage an der ersten Steilflanke 6.

Der zweite Drehmomentübertragungskörper 2 besitzt eine Abstützfläche 18, an der sich ein unteres Ende eines als Stahlwendelgang-Druckfeder ausgebildeten Federelementes 8 abstützt. Beim Ausführungsbeispiel wird die Abstützfläche 18 durch eine Metallscheibe 19 ausgebildet, die in einer Höhlung des Drehmomentübertragungskörpers 2 einliegt.

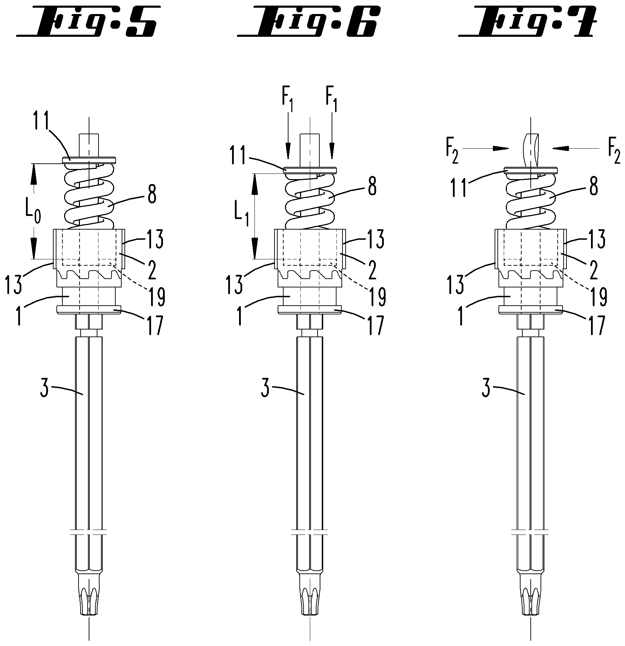

Der Schaft 3 erstreckt sich durch das Federelement 8 und überragt das nach oben weisende zweite Ende des Federelementes 8 und eine auf dem oberen Ende des Federelementes 8 aufliegende, ein Zwischenglied 11 bildende Unterlegscheibe, durch deren Loch 12 sich der Schaft 3 erstreckt. In diesem, in der Figur 5 dargestellten Zustand besitzt das Federelement 8, welches nicht kraftbeaufschlagt ist, seine Neutrallänge L0. Der sich durch das Federelement 8 erstreckende Schaftabschnitt 3' des Schaftes 3 besitzt bevorzugt ein Rundprofil.

In einem weiteren Montageschritt, der in der Figur 6 dargestellt ist, wird das obere Ende des Federelementes 8 beziehungsweise das darauf liegende von einer Unterlegscheibe gebildete Zwischenglied 11 mit einer vorbestimmten Kraft F1 in Achsrichtung beaufschlagt. Dies führt zu einer Längenverkürzung des Federelementes 8 auf eine Kalibrierlänge L1.

Anstelle eines Zwischengliedes 11 können auch mehrere Zwischenglieder 11 übereinander angeordnet sein. Die Zwischenglieder 11 bilden dann Gleitlagerplatten, die bei einer Drehbewegung des Schaftes gegenüber dem Federelement 8 gegeneinander abgleiten können.

In dem darauf folgenden, in der Figur 7 dargestellten Verfahrensschritt wird das Federelement 8 in dieser Länge L1 fixiert. Dies erfolgt durch Aufbringen von radialen Kräften F2 auf das, das obere Ende des Federelementes 8 beziehungsweise die Unterlegscheibe überragende Ende des Schaftes 3, welches ein Widerlager 9 ausbildet. Aufgrund dieser Kraftbeaufschlagung, die unter Verwendung von zwei Druckbacken erfolgen kann, wird das freie Ende des Schaftes 3 zum Widerlager 9 verformt. Dabei bilden sich Schultern aus, an denen sich die Unterlegscheibe abstützen kann. Es entstehen zwei sich gegenüberliegende Breitseitenflächen 14, deren Abstand voneinander geringer ist, als der Durchmesser des Schaftes, so dass ein Materialfluss zu den Schmalseiten 15 hin erfolgt, die die Schultern ausbilden, an denen sich das Zwischenglied 11 abstützt.

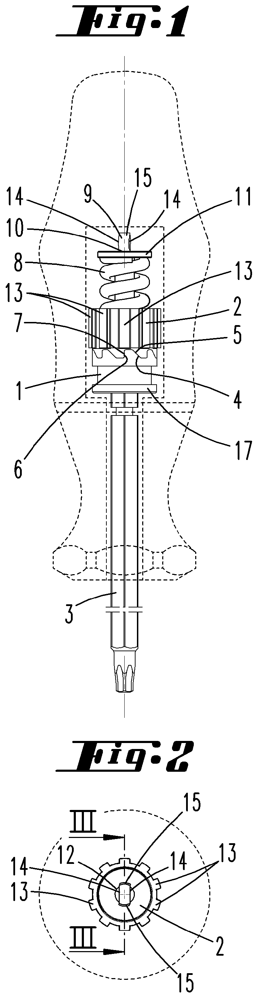

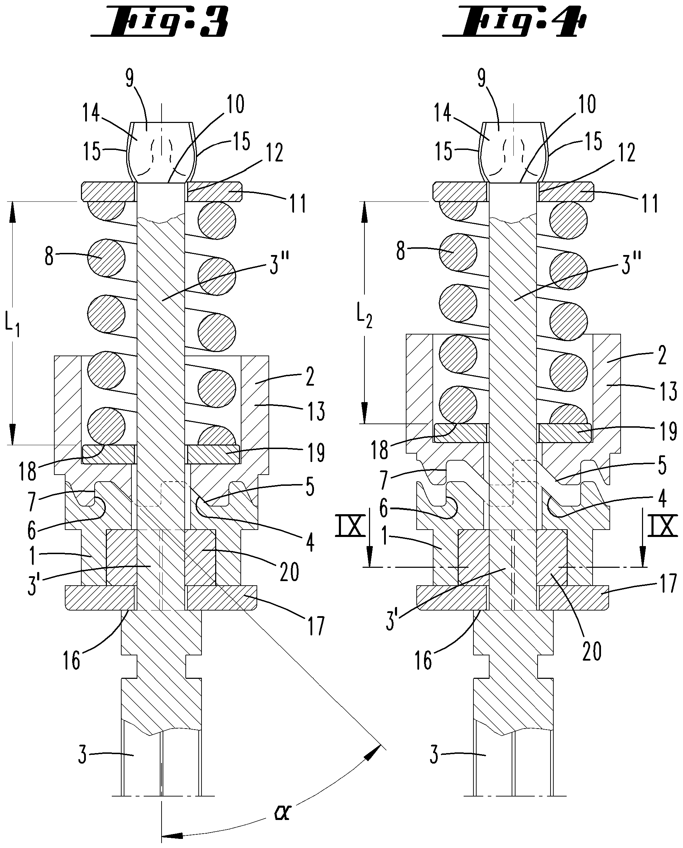

Es ist dann der in den Figuren 1 und 3 dargestellte Gebrauchszustand erreicht. Wird in diesem Zustand der erste Drehmomentübertragungskörper 1 ortsfest gehalten und auf den zweiten Drehmomentübertragungskörper 2 ein Drehmoment im Uhrzeigersinn aufgebracht, so gleiten die beiden Schrägflanken 4, 5 aneinander ab. Wird ein Drehmoment in Gegenrichtung aufgebracht, so stützen sich die beiden Steilflanken 6, 7 aneinander ab.

Beim Aufbringen des Drehmomentes im Uhrzeigersinn können die beiden Schrägflanken 4, 5 bis in die in der Figur 4 dargestellte Stellung aneinander abgleiten. In dieser Position haben sich die beiden Drehmomentübertragungskörper 1, 2 etwa um die Höhe des Drehmomentübertragungszahnes voneinander beabstandet. Das Federelement 8 ist von der Länge L1 auf die verkürzte Länge L2 komprimiert worden. Diese Position entspricht einem Auslösedrehmoment, welches dem Grenzdrehmoment entspricht, das mit der Drehmomentbegrenzungseinrichtung übertragbar ist.

In einer Variante des Kalibrierens der Drehmomentbegrenzungseinrichtung kann in dem in der Figur 6 dargestellten Schritt eine größere Kraft F1 verwendet werden die das Federelement 8 auf die Länge L2 komprimiert. Die Anschlagstufe des Widerlagers 9 wird dann in einem definierten axialen Abstand zur Oberkante der Unterlegscheibe 11 beziehungsweise des Federelementes 8 erzeugt, wobei dieser axiale Abstand dem maximalen axialen Verlagerungsweg des zweiten Drehmomentübertragungskörpers 2 gegenüber dem ersten Drehmomentübertragungskörper 1 entspricht, also in etwa der Höhe des Drehmomentübertragungszahnes.

In einer weiteren Variante des Kalibrierens kann die in der in der Figur 6 dargestellte Beaufschlagung des Federelementes 8 mit einer beispielsweise von einem Normgewicht erzeugten Kalibrierkraft F1 auch in dem in Figur 4 dargestellten Zustand erfolgen, wenn sich die Kalibrierzähne der beiden Drehmomentübertragungskörper 1, 2 aneinander abstützen.

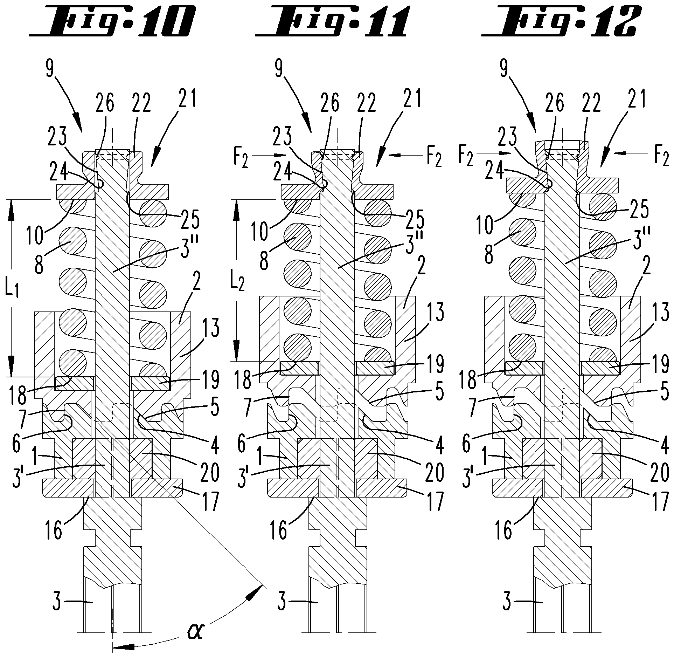

Bei den in den Figuren 10 bis 12 dargestellten weiteren Ausführungsbeispiel ist der Schaft 3 aus einem gehärteten Werkstoff gefertigt und bildet das Schraubwerkzeug aus. Es kann aber auch vorgesehen sein, dass am freien Ende des Schaftes 3 ein Futter angeordnet ist zur Aufnahme eines Schraubwerkzeuges.

Wesentlich ist, dass sich das Federelement 8 an einem tellerförmigen Abschnitt eines Abstützelementes 21 abstützt. Das im Wesentlichen rotationssymmetrische Abstützelement 21 besitzt einen scheibenförmigen Abschnitt, der die Abstützschulter 10 ausbildet. Von diesem Abschnitt ragt in Axialrichtung ein Hals 22 ab, dessen Innendurchmesser geringfügig größer ist, als der Außendurchmesser des Schaftes 3. Dieser Hals 22 ist auf das Ende des Schaftes 3 aufgesteckt und wird in der zuvor beschriebenen Weise zur Kalibrierung der Drehmomentbegrenzungseinrichtung mit einer Axialkraft beaufschlagt. Dabei können die Zähne der beiden Drehmomentübertragungskörper 1, 2 ineinandergreifen, wie es die Figur 10 zeigt. Es ist aber auch möglich, dass sich die Köpfe der Zähne der Drehmomentübertragungskörper 1, 2 aufeinander abstützen, wie es die Figuren 11 und 12 zeigen.

Die irreversible Fixierung des Abstützelementes 21 erfolgt durch eine quer zur Erstreckungsrichtung des Schaftes aufgebrachte Verformungskraft. Die Verformungskraft wird durch nicht dargestellte Verformungsbacken erzeugt. Es können mehrere, sternförmig angeordnete Backen vorgesehen sein, die in radialer Richtung aufeinander zu fahren, um den Hals 22 am Ende des Schaftes zu befestigen.

Das Ende des Schaftes besitzt eine Aussparung 23, die eine Konusfläche 24 aufweist, die an eine Stufe 25 angrenzt. In diese Aussparung 23 wird das bevorzugt weichere Material als das Material des Schaftes 3 des Halses 22 eingepresst.

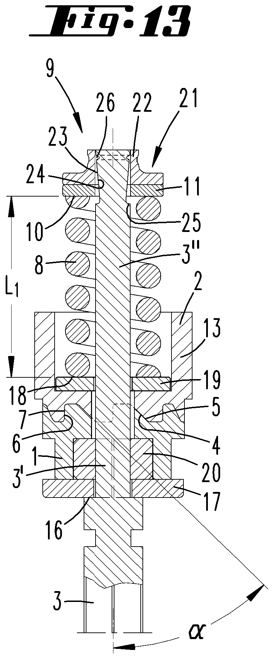

Bei dem in der Figur 13 dargestellten Ausführungsbeispiel ist zwischen dem Ende des Federelementes 8 und dem Abstützelement 21 ein Zwischenglied 11 in Form einer Gleitlagerscheibe angeordnet. Die Gleitlagerscheibe 11 ist als Unterlegscheibe ausgebildet und kann aus Stahl, Kunststoff, Keramik, Graphit, einem Sinterwerkstoff oder einer Nichteisenlegierung gefertigt sein. Es können auch mehrere derartige Zwischenglieder 11 übereinander angeordnet sein. Die Aufgabe dieses Zwischengliedes 11 ist die Reibminimierung. Der Schaft 3 kann sich bei der Überschreitung des Grenzdrehmomentes gegenüber dem Federelement 8 verdrehen. Die zum Abstützelement 21 weisende Breitseitenfläche des Zwischengliedes 11 bildet eine Gleitreibungsfläche, die sich bei einer Drehung gegenüber einer Breitseitengegenfläche des Abstützelementes 21 verdreht. Die Flächenreibung der aufeinanderliegenden Breitseitenflächen wird durch eine reibvermindernde Oberflächeneigenschaft vermindert. Die Erfindung betrifft somit auch eine Vorrichtung, bei der zwischen Federelement 8 und Abstützelement 21 eine Gleitscheibe 11 angeordnet ist, die mit einer Gleitfläche an einer Gegengleitfläche des Abstützelementes 21 anliegt.

Die vorstehenden Ausführungen dienen der Erläuterung der von der Anmeldung insgesamt erfassten Erfindungen, die den Stand der Technik zumindest durch die folgenden Merkmalskombinationen jeweils auch eigenständig weiterbilden, wobei zwei, mehrere oder alle dieser Merkmalskombinationen auch kombiniert sein können, nämlich:

Eine Drehmomentbegrenzungseinrichtung, die dadurch gekennzeichnet ist, dass das Widerlager 9 materialeinheitlich vom Schaft 3 oder von einem am Schaft 3 irreversibel befestigten Abstützelement 11, 21 gebildet ist.

Eine Drehmomentbegrenzungseinrichtung, die dadurch gekennzeichnet ist, dass das Abstützelement 21 unter Ausbildung einer Formschlussverbindung einhergehend mit einer plastischen Verformung am Schaft 3 befestigt ist.

Eine Drehmomentbegrenzungseinrichtung, die dadurch gekennzeichnet ist, dass das Abstützelement 21 eine zum Federelement 8 weisende Breitseite eines tellerförmigen Abschnittes ausbildet und einen von der Breitseite wegweisenden Hals 22, der unter Ausbildung einer plastischen Verformung mit dem Schaft 3 verbunden ist.

Eine Drehmomentbegrenzungseinrichtung, die dadurch gekennzeichnet ist, dass der Schaft 3 eine Aussparung 23 aufweist, in die Material des Abstützelementes 21 oder eines Halses 22 des Abstützelementes 21 eingepresst ist.

Eine Drehmomentbegrenzungseinrichtung, die dadurch gekennzeichnet ist, dass die Aussparung 23 eine Konusfläche 24 und/oder eine Stufe 25 aufweist.

Eine Drehmomentbegrenzungseinrichtung, die dadurch gekennzeichnet ist, dass sich das Federelement 8 unmittelbar oder unter Zwischenlage eines Zwischengliedes 11 am Widerlager 9 abstützt, wobei insbesondere vorgesehen ist, dass das Zwischenglied 11 eine Unterlegscheibe ist und/oder dass sich das Federelement 8 unter Zwischenlage eines zweiten Zwischengliedes 19, insbesondere einer Unterlegscheibe 19 an einem der Drehmomentübertragungskörper 1, 2 abstützt.

Eine Drehmomentbegrenzungseinrichtung, die dadurch gekennzeichnet ist, dass die Drehmomentübertragungskörper 1 sich mittels einer Hubeinrichtung bei der Relativdrehung voneinander beabstanden, wobei die Hubeinrichtung insbesondere von einer ersten Schrägflanke 4 eines ersten Drehmomentübertragungskörpers 1, der fest mit dem Schaft 3 verbunden ist, und einer zweiten Schrägflanke 5 eines zweiten Drehmomentübertragungskörpers 2, der gegenüber dem Schaft 3 drehbar ist, gebildet ist, wobei insbesondere vorgesehen ist, dass der zweite Drehmomentübertragungskörper 2 drehfest aber axial verschieblich einem Griff zugeordnet ist, wobei insbesondere vorgesehen ist, dass die Drehmomentbegrenzungseinrichtung in einer Höhlung des Griffs angeordnet ist, in der sich der zweite Drehmomentübertragungskörper 2 in Achsrichtung verlagern kann und/oder dass der zweite Drehmomentübertragungskörper 2 als Folge einer Verrippung in einer Höhlung des Griffs axial verschieblich ist.

Eine Drehmomentbegrenzungseinrichtung, die gekennzeichnet ist durch eine in gleitreibender Flächenanlage an einer Gegenfläche des Abstützelementes 11 anliegende Gleitlagerscheibe 11, an der sich das Federelement 8 abstützt.

Ein Verfahren, das dadurch gekennzeichnet ist, dass zwei bei einer Relativdrehung um die Achse A in Richtung nämlicher Achse A relativ zueinander gegen die Rückstellkraft eines vorgespannten Federelementes 8 verlagerbaren Drehmomentübertragungskörpern 1, 2, wobei das im kraftunbeaufschlagten Zustand eine Neutrallänge L0 aufweisende Federelement 8 durch axiale Beaufschlagung mit einer vorbestimmten externen Kraft F1 von der Neutrallänge L0 auf eine kalibrierte Länge L1, L2 gebracht wird, in der ein Widerlager 9 irreversibel fixiert wird, dadurch gekennzeichnet, dass die Länge L1, L2 durch eine plastische Verformung fixiert wird.

Ein Verfahren, das dadurch gekennzeichnet ist, dass sowohl ein Lager 17 als auch das Widerlager 9, zwischen denen das Federelement 8 eingespannt ist, irreversibel an einem Schaft 3 befestigt sind.

Ein Verfahren, das dadurch gekennzeichnet ist, dass das Widerlager 9 materialeinheitlich vom Schaft 3 oder von einem am Schaft 3 irreversibel befestigten Abstützelement 11, 21 ausgebildet wird.

Ein Verfahren, das dadurch gekennzeichnet ist, dass beim Fixieren des Widerlagers 9 sich die Drehmomentübertragungszähne der Drehmomentübertragungskörper 1, 2 aneinander abstützen.

Eine Produktionscharge, die dadurch gekennzeichnet ist, dass die Drehmomentbegrenzungseinrichtung gemäß einem der Ansprüche 1 bis 7 ausgebildet ist und die vorbestimmte Kraft F1 bei allen Drehmomentbegrenzungseinrichtungen dieselbe ist.

Eine Produktionscharge, die dadurch gekennzeichnet, dass die Drehmomentbegrenzungseinrichtung nach einem Verfahren gemäß einem der Ansprüche 8 bis 11 ausgebildet ist, wobei die vorbestimmte Kraft F1 bei allen Drehmomentbegrenzungseinrichtungen dieselbe ist.

Alle offenbarten Merkmale sind (für sich, aber auch in Kombination untereinander) erfindungswesentlich.

### Liste der Bezugszeichen

| | | | |
|---|---|---|---|
| 1 | Drehmomentübertragungskörper | 22 | Hals |
| 2 | Drehmomentübertragungskörper | 23 | Aussparung |
| 3 | Schaft | 24 | Konusfläche |
| 3' | Sechskantabschnitt | 25 | Stufe |
| 3" | Rundabschnitt | 26 | Rille |
| 4 | Schrägflanke | | |
| 5 | Schrägflanke | | |
| 6 | Steilflanke | | |
| 7 | Steilflanke | A | Drehachse |
| 8 | Federelement | F1 | Kraft |
| 9 | Widerlager | F2 | Kraft |
| 10 | Abstützschulter | L0 | Neutrallänge |
| 11 | Zwischenglied (Unterlegscheibe) | L1 | Länge |
| 12 | Loch | | |
| 13 | Rippe | | |
| 14 | Breitseite | α | Winkel |
| 15 | Schmalseite | | |
| 16 | Stufe | | |
| 17 | Unterlegscheibe | | |
| 18 | Abstützfläche | | |
| 19 | Zwischenglied (Unterlegscheibe/Gleitlagerscheibe) | | |
| 20 | Sechskant-Mitnehmerscheibe | | |
| 21 | Abstützelement | | |

## Patentansprüche

1. Drehmomentbegrenzungseinrichtung mit zwei Drehmomentübertragungskörpern (1, 2), von denen zumindest einer bei einer Relativdrehung um eine Achse (A) in Richtung nämlicher Achse (A) gegenüber dem anderen Drehmomentübertragungskörper (2, 1) gegen die Rückstellkraft eines mit einer Vorspannung zwischen einem Lager (17) und einem Widerlager (9) eingespannten Federelementes (8) entlang eines sich in der Achse (A) erstreckenden Schaftes (3) verlagerbar ist, wobei die Vorspannung des Federelementes (8) bei der Fertigung der Drehmomentbegrenzungseinrichtung irreversibel durch die axiale Lage des Widerlagers (9) eingestellt ist, wobei das Widerlager (9) materialeinheitlich vom Schaft (3) oder von einem am Schaft (3) irreversibel befestigten Abstützelement (11, 21) gebildet ist, **dadurch gekennzeichnet, dass** das Widerlager (9) bei der Montage derart mit dem Schaft (3) verbindbar ist, dass die axiale Lage des Widerlagers (9) durch Beaufschlagen des Federelementes (8) mit einer vorbestimmten externen Kraft (F1) vor der Befestigung des Widerlagers (9) am Schaft (3) vorgegeben werden kann.

2. Drehmomentbegrenzungseinrichtung nach Anspruch 1, **dadurch gekennzeichnet, dass** das Abstützelement (21) unter Ausbildung einer Formschlussverbindung einhergehend mit einer plastischen Verformung am Schaft (3) befestigt ist.

3. Drehmomentbegrenzungseinrichtung nach einem der vorhergehenden Ansprüche, **dadurch gekennzeichnet, dass** das Abstützelement (21) eine zum Federelement (8) weisende Breitseite eines tellerförmigen Abschnittes ausbildet und einen von der Breitseite wegweisenden Hals (22), der unter Ausbildung einer plastischen Verformung mit dem Schaft (3) verbunden ist.

4. Drehmomentbegrenzungseinrichtung nach Anspruch 2, **dadurch gekennzeichnet, dass** der Schaft (3) eine Aussparung (23) aufweist, in die Material des Abstützelementes (21) oder eines Halses (22) des Abstützelementes (21) eingepresst ist.

5. Drehmomentbegrenzungseinrichtung nach Anspruch 4, **dadurch gekennzeichnet, dass** die Aussparung (23) eine Konusfläche (24) und/oder eine Stufe (25) aufweist.

6. Drehmomentbegrenzungseinrichtung nach einem der vorhergehenden Ansprüche, **dadurch gekennzeichnet, dass** sich das Federelement (8) unmittelbar oder unter Zwischenlage eines Zwischengliedes (11) am Widerlager (9) abstützt, wobei insbesondere vorgesehen ist, dass das Zwischenglied (11) eine Unterlegscheibe ist und/oder dass sich das Federelement (8) unter Zwischenlage eines zweiten Zwischengliedes (19), insbesondere einer Unterlegscheibe (19) an einem der Drehmomentübertragungskörper (1, 2) abstützt.

7. Drehmomentbegrenzungseinrichtung nach einem der vorhergehenden Ansprüche, **dadurch gekennzeichnet, dass** die Drehmomentübertragungskörper (1) sich mittels einer Hubeinrichtung bei der Relativdrehung voneinander beabstanden, wobei die Hubeinrichtung insbesondere von einer ersten Schrägflanke (4) eines ersten Drehmomentübertragungskörpers (1), der fest mit dem Schaft (3) verbunden ist, und einer zweiten Schrägflanke (5) eines zweiten Drehmomentübertragungskörpers (2), der gegenüber dem Schaft (3) drehbar ist, gebildet ist, wobei insbesondere vorgesehen ist, dass der zweite Drehmomentübertragungskörper (2) drehfest aber axial verschieblich einem Griff zugeordnet ist, wobei insbesondere vorgesehen ist, dass die Drehmomentbegrenzungseinrichtung in einer Höhlung des Griffs angeordnet ist, in der sich der zweite Drehmomentübertragungskörper (2) in Achsrichtung verlagern kann und/oder dass der zweite Drehmomentübertragungskörper (2) als Folge einer Verrippung in einer Höhlung des Griffs axial verschieblich ist.

8. Drehmomentbegrenzungseinrichtung nach einem der vorhergehenden Ansprüche, **gekennzeichnet durch** eine in gleitreibender Flächenanlage an einer Gegenfläche des Abstützelementes (11) anliegende Gleitlagerscheibe (11), an der sich das Federelement (8) abstützt.

9. Verfahren zum Kalibrieren einer Drehmomentbegrenzungseinrichtung, nach einem der vorhergehenden Ansprüche, mit zwei bei einer Relativdrehung um die Achse (A) in Richtung nämlicher Achse (A) relativ zueinander gegen die Rückstellkraft eines vorgespannten Federelementes (8) verlagerbaren Drehmomentübertragungskörpern (1, 2), wobei das im kraftunbeaufschlagten Zustand eine Neutrallänge (L0) aufweisende Federelement (8) durch axiale Beaufschlagung mit einer vorbestimmten externen Kraft (F1) von der Neutrallänge (L0) auf eine kalibrierte Länge (L1, L2) gebracht wird, in der ein Widerlager (9) irreversibel fixiert wird, **dadurch gekennzeichnet, dass** die Länge (L1, L2) durch eine plastische Verformung fixiert wird.

10. Verfahren nach Anspruch 9, **dadurch gekennzeichnet, dass** sowohl ein Lager (17) als auch das Widerlager (9), zwischen denen das Federelement (8) eingespannt ist, irreversibel an einem Schaft (3) befestigt sind.

11. Verfahren nach einem der Ansprüche 9 oder 10, **dadurch gekennzeichnet, dass** das Widerlager (9) materialeinheitlich vom Schaft (3) oder von einem am Schaft (3) irreversibel befestigten Abstützelement (11, 21) ausgebildet wird.

12. Verfahren nach einem der Ansprüche 9 bis 11, **dadurch gekennzeichnet, dass** beim Fixieren des Widerlagers (9) sich die Drehmomentübertragungszähne der Drehmomentübertragungskörper (1, 2) aneinander abstützen.

13. Aus einer Vielzahl von Schraubwerkzeugen mit jeweils einer Drehmomentbegrenzungseinrichtung bestehende Produktionscharge von Drehmomentübertragungswerkzeugen, **dadurch gekennzeichnet, dass** die Drehmomentbegrenzungseinrichtung gemäß einem der Ansprüche 1 bis 8 ausgebildet ist und die vorbestimmte Kraft (F1) bei allen Drehmomentbegrenzungseinrichtungen dieselbe ist.

14. Aus einer Vielzahl von Schraubwerkzeugen mit jeweils einer Drehmomentbegrenzungseinrichtung bestehende Produktionscharge von Drehmomentübertragungswerkzeugen, **dadurch gekennzeichnet, dass** die Drehmomentbegrenzungseinrichtung nach einem Verfahren gemäß einem der Ansprüche 9 bis 12 gefertigt ist, wobei die vorbestimmte Kraft (F1) bei allen Drehmomentbegrenzungseinrichtungen dieselbe ist.

15. Verfahren nach einem der Ansprüche 9 bis 12, **dadurch gekennzeichnet, dass** das Abstützelement (11, 21) die durch Beaufschlagung des Federelementes (8) mit einer vordefinierten Kraft kalibrierte Länge des Federelementes (8) lagefixiert.

## Claims

1. A torque-limiting device having two torque transfer bodies (1, 2), whereof at least one is displaceable during a relative rotation about an axis (A) in the direction of said axis (A) with respect to the other torque transfer body (2, 1) against the restoring force of a spring element (8) clamped with pretensioning between a bearing (17) and an abutment (9) along a shaft (3) extending in the axis (A), wherein the pretensioning of the spring element (8) is set irreversibly by the axial position of the abutment (9) during the production of the torque-limiting device, **characterised in that** the abutment (9) can be connected to the shaft (3) during assembly in such a way that the axial position of the abutment (9) can be predetermined by applying a predetermined external force (F1) to the spring element (8) before the abutment (9) is fastened to the shaft (3).

2. The torque-limiting device according to claim 1, **characterised in that** the support element (21) is fastened to the shaft (3) thereby forming a form-fit connection in conjunction with a plastic deformation.

3. The torque-limiting device according to one of the preceding claims, **characterised in that** the support element (21) forms a wide side of a saucer-shaped section that points towards the spring element (8) and a neck (22), which points away from the wide side and which is connected to the shaft (3) thereby forming a plastic deformation.

4. The torque-limiting device according to claim 2, **characterised in that** the shaft (3) comprises a recess (23), into which material of the support element (21) or of a neck (22) of the support element (21) is pressed.

5. The torque-limiting device according to claim 4, **characterised in that** the recess (23) has a conical surface (24) and/or a step (25).

6. The torque-limiting device according to one of the preceding claims, **characterised in that** the spring element (8) is supported on the abutment (9) directly or with the interposition of an intermediate element (11), it being provided in particular that the intermediate element (11) is a washer and/or that the spring element (8) is supported on one of the torque transfer bodies (1, 2) with the interposition of a second intermediate member (19), in particular a washer (19).

7. The torque-limiting device according to one of the preceding claims, **characterised in that** the torque transfer bodies (1) move away from one another during the relative rotation by means of a lifting device, wherein the lifting device is formed in particular by a first oblique flank (4) of a first torque transfer body (1), which is fixedly connected to the shaft (3), and a second oblique flank (5) of a second torque transfer body (2), which is rotatable with respect to the shaft (3), or that a second torque transfer body (2) is assigned rotationally fixed but axially displaceable to a handle, or that the torque-limiting device is arranged in a cavity of the handle, in which a second torque transfer body (2) can move in the axial direction or that a second torque transfer body (2) is axially displaceable as a consequence of ribbing in a cavity of the handle.

8. The torque-limiting device according to one of the preceding claims, **characterised by** a sliding bearing disc (11) lying in sliding friction surface contact against a counter-surface of the support element (11), on which sliding bearing disc the spring element (8) is supported.

9. A method for calibrating a torque-limiting device, according to one of the preceding claims, with two torque transfer bodies (1, 2), which are displaceable during a relative rotation about the axis (A) in the direction of said axis (A) relative to one another against the restoring force of a pretensioned spring element (8), wherein the spring element (8) having a neutral length (L0) in the state when the force is not applied is brought from the neutral length (L0) to a calibrated length (L1, L2) by axial application with a predefined external force (F1), in which calibrated length an abutment (9) is irreversibly fixed, **characterised in that** the length (L1, L2) is fixed by a plastic deformation.

10. The method according to claim 9, **characterised in that** both a bearing (17) and the abutment (9), between which the spring element (8) is clamped, are irreversibly fastened to a shaft (3).

11. The method according to one of the claims 9 or 10, **characterised in that** the abutment (9) is formed uniformly in terms of material by the shaft (3) or by a support element (11, 21) fastened irreversibly to the shaft (3).

12. The method according to one of the claims 9 to 11, **characterised in that** the torque transfer teeth of the torque transfer bodies (1, 2) rest against one another when the abutment (9) is fixed.

13. A production batch of torque transfer tools which comprises a plurality of screwdrivers with in each case a torque-limiting device, **characterised in that** the torque-limiting device is constituted according to any one of claims 1 to 8 and the predefined force (F1) is the same in the case of all the torque-limiting devices.

14. A production batch of torque transfer tools which comprises a plurality of screwdrivers with in each case a torque-limiting device, **characterised in that** the torque-limiting device is produced according to a method according to one of claims 9 to 12, wherein the predefined force (F1) is the same in the case of all the torque-limiting devices.

15. The torque-limiting device according to one of the claims 9 to 12, **characterised in that** the support element (11, 21) fixes in position the length of the spring element (8) calibrated by application of a predefined force to the spring element (8).

## Revendications

1. Dispositif limiteur d'un couple de rotation, pourvu de deux corps de transmission (1, 2) d'un couple de rotation, dont au moins l'un est déplaçable lors d'une rotation relative autour d'un axe (A) dans la direction dudit axe (A) par rapport à l'autre corps de transmission (2, 1) d'un couple de rotation, à l'encontre de la force de rappel d'un élément à ressort (8) enserré avec une précontrainte entre un palier (17) et un contre-palier (9) le long d'une tige (3) s'étendant dans l'axe (A), la précontrainte de l'élément à ressort (8) lors de la fabrication du dispositif limiteur d'un couple de rotation étant réglée de manière irréversible par la position axiale du contre-palier (9), le contre-palier (9) étant formé dans la même matière que la tige (3) ou un élément de soutien (11, 21) fixé de manière irréversible sur la tige (3), **caractérisé en ce que**, lors du montage, le contre-palier (9) est susceptible d'être assemblé avec la tige (3), de telle sorte que la position axiale du contre-palier (9) puisse être prédéterminée par la sollicitation de l'élément à ressort (8) avec une force externe (F1) prédéfinie avant la fixation du contre-palier (9) sur la tige (3).

2. Dispositif limiteur d'un couple de rotation selon la revendication 1, **caractérisé en ce que** l'élément de soutien (21) est fixé sur la tige (3) en formant un assemblage par complémentarité de forme accompagné d'une déformation plastique.

3. Dispositif limiteur d'un couple de rotation selon l'une quelconque des revendications précédentes, **caractérisé en ce que** l'élément de soutien (21) forme un côté large dirigé vers l'élément à ressort (8) d'un segment en forme de coupelle et un col (22) dirigé en éloignement du côté large, qui est assemblé avec la tige (3) en formant une déformation plastique.

4. Dispositif limiteur d'un couple de rotation selon la revendication 2, **caractérisé en ce que** la tige (3) comporte une encoche (23) dans laquelle est pressée de la matière de l'élément de soutien (21) ou d'un col (22) de l'élément de soutien (21).

5. Dispositif limiteur d'un couple de rotation selon la revendication 4, **caractérisé en ce que** l'encoche (23) comporte une surface conique (24) et/ou un gradin (25).

6. Dispositif limiteur d'un couple de rotation selon l'une quelconque des revendications précédentes, **caractérisé en ce que** l'élément à ressort (8) est soutenu directement ou avec interposition d'un organe intermédiaire (11) sur le contre-palier (9), étant notamment prévu que l'organe intermédiaire (11) soit une rondelle et/ou que l'élément à ressort (8) soit soutenu sous interposition d'un deuxième organe intermédiaire (19), notamment d'une rondelle (19) sur l'un des corps de transmission (1, 2) d'un couple de rotation.

7. Dispositif limiteur d'un couple de rotation selon l'une quelconque des revendications précédentes, **caractérisé en ce que** les corps de transmission (1) d'un couple de rotation s'écartent l'un de l'autre au moyen d'un dispositif de levage lors de la rotation relative, le dispositif de levage étant formé notamment d'un premier flanc oblique (4) d'un premier corps de transmission (1) d'un couple de rotation, qui est fixement assemblé avec la tige (3) et d'un deuxième flanc oblique (5) d'un deuxième corps de transmission (2) d'un couple de rotation, qui est rotatif par rapport à la tige (3), étant notamment prévu qu'au deuxième corps de transmission (2) d'un couple de rotation, une poignée soit associée de manière solidaire en rotation, mais coulissante en direction axiale, étant notamment prévu que le dispositif limiteur d'un couple de rotation soit placé dans une cavité de la poignée, dans laquelle le deuxième corps de transmission (2) d'un couple de rotation peut se déplacer et/ou que le corps de transmission (2) d'un couple de rotation soit coulissant en direction axiale dans une cavité de la poignée, du fait d'un nervurage.

8. Dispositif limiteur d'un couple de rotation selon l'une quelconque des revendications précédentes, **caractérisé par** un disque de palier lisse (11) adjacent en appui plan en friction par coulissement sur une contre-surface de l'élément de soutien (11) sur lequel est soutenu l'élément à ressort (8).

9. Procédé, destiné à étalonner un dispositif limiteur d'un couple de rotation, selon l'une quelconque des revendications précédentes, pourvu de deux corps de transmission (1, 2) d'un couple de rotation, déplaçables l'un par rapport à l'autre à l'encontre de la force de rappel d'un élément à ressort (8) précontraint, lors d'une rotation relative autour de l'axe (A), dans la direction dudit axe (A), l'élément à ressort (8) qui présente une longueur neutre (L0) à l'état non sollicité par une force étant amené par sollicitation axiale avec une force externe (F1) prédéfinie de la longueur neutre (L0) à une longueur étalonnée (L1, L2) dans laquelle un contre-palier (9) est fixé de manière irréversible, **caractérisé en ce que** la longueur (L1, L2) est fixée par une déformation plastique.

10. Procédé selon la revendication 9, **caractérisé en ce qu'**aussi bien un palier (17) qu'également le contre-palier (9), entre lesquels l'élément à ressort (8) est enserré sont fixés de manière irréversible sur une tige (3).

11. Procédé selon l'une quelconque des revendications 9 ou 10, **caractérisé en ce que** le contre-palier (9) est dans la même matière que la tige (3) ou un élément de soutien (11, 21) fixé de manière irréversible sur la tige (3).

12. Procédé selon l'une quelconque des revendications 9 à 11, caractérisé en ce lors de la fixation du contre-palier (9), les dents de transmission d'un couple de rotation des corps de transmission (1, 2) d'un couple de rotation se soutiennent les unes sur les autres.

13. Charge de production d'outils de transmission d'un couple de rotation, constituée d'une pluralité d'outils de vissage pourvus chacun d'un dispositif limiteur d'un couple de rotation, **caractérisée en ce que** le dispositif limiteur d'un couple de rotation est conçu selon l'une quelconque des revendications 1 à 8 et la force prédéfinie (F1) est la même pour tous les dispositifs limiteurs d'un couple de rotation.

14. Charge de production d'outils de transmission d'un couple de rotation, constituée d'une pluralité d'outils de vissage pourvus chacun d'un dispositif limiteur d'un couple de rotation, **caractérisée en ce que** le dispositif limiteur d'un couple de rotation est fabriqué d'après un procédé selon l'une quelconque des revendications 9 à 12, la force prédéfinie (F1) étant la même pour tous les dispositifs limiteurs d'un couple de rotation.

15. Procédé selon l'une quelconque des revendications 9 à 12, **caractérisé en ce que** l'élément de soutien (11, 21) fixe en position la longueur de l'élément à ressort (8) étalonnée par sollicitation de l'élément à ressort (8) avec une force prédéfinie.
